# EUROPÄISCHE PATENTSCHRIFT

(11) **EP 0 797 097 B1**
(45) Veröffentlichungstag und Bekanntmachung des Hinweises auf die Patenterteilung: **12.12.2001**
(21) Anmeldenummer: 96104283.5
(22) Anmeldetag: 18.03.1996
(51) Int. Cl.: G01N 33/543, B01L 3/14, G01N 33/80

(54) **Partikel-Immunoassay mit kompakter Matrix**
Particle immuno assay with compact matrix
Essai immunologique particule avec une matrice compacte

(43) Veröffentlichungstag der Anmeldung: 24.09.1997
(73) Patentinhaber: STIFTUNG FÜR DIAGNOSTISCHE FORSCHUNG, CH-1785 Cressier sur Morat (CH)
(72) Erfinder: Josef, Dieter, 1785 Cressier (CH); Greber, Suzanne, 3037 Herrenschwanden (CH); Adam, Jean, 1884 Villars (CH)
(74) Vertreter: Weiss, Wolfgang, Dipl.-Chem. Dr.

(56) Entgegenhaltungen:
- EP-A- 0 143 412
- EP-A- 0 194 212
- EP-A- 0 305 337
- EP-A- 0 485 228
- WO-A-92/05440
- FR-A- 2 445 528

## Beschreibung

Die vorliegende Erfindung betrifft ein Verfahren zum Nachweis eines Analyten in einer Probeflüssigkeit durch Agglutination, wobei man die Probeflüssigkeit mit einem Agglutinationsreagenz in Kontakt bringt und eine Reaktion zwischen dem Analyten und dem Agglutinationsreagenz bestimmt. Weiterhin werden Reaktionsgefäße und Reagenzien zur Durchführung des erfindungsgemäßen Verfahrens offenbart.

Methoden zum Nachweis von Analyten durch Agglutination sind bekannt. So wird in EP-B-0 194 202 ein Verfahren zum Nachweis von Erythrozyten-Agglutinationen offenbart, bei dem ein Gemisch von Serum und Erythrozyten nach Inkubation einem gegebenenfalls Antikörper enthaltenden Gelmedium zugegeben wird und dieses Gemisch Sedimentationsbedingungen unterworfen wird, die die Bestimmung einer Erythrozyten-Agglutination ermöglichen.

EP-A-0 305 337 offenbart ein Verfahren zum Nachweis von Antikörpern bzw. Antigenen durch optische Sichtbarmachung von Komplexen trägergebundener Antigene mit Antikörpern in wässrigem Medium, wobei eine Lösung, die einen Antikörper bzw. ein Antigen enthält, mit einem trägergebundenen Antigen bzw. Antikörper in Kontakt gebracht wird, wobei eine Aufschlämmung oder Suspension von inerten Partikeln vor, während oder nach dieser Reaktion zugesetzt wird und anschließend die Mischung der Gravitation ausgesetzt wird, wobei bei der Bildung eines Antigen-Antikörper-Komplexes dieser im stark positiven Fall auf dem Sediment der inerten Partikel liegt und im schwach positiven Fall innerhalb der inerten Partikel vorhanden ist und bei Abwesenheit eines Antigen-Antikörper-Komplexes, d.h. im negativen Fall, die trägergebundenen Antikörper bzw. Antigene unter den sedimentierten inerten Partikeln liegen. Als inerte Partikel können beispielsweise Dextrosepolymere oder Glaskügelchen verwendet werden.

Die Verwendung eines Gelmediums bzw. einer partikelförmigen Matrix kann jedoch eine Reihe von Nachteilen führen. So ist die Schicht aus inerten Partikeln mechanisch nicht stabil, die Oberfläche verschiebt sich bei horizontaler Lagerung oder verspritzt bei Erschütterungen, so daß unter Umständen zweifelhafte oder sogar falsche Resultate vorkommen können. Weiterhin müssen die inerten Partikel mit höchster Präzision hergestellt werden, da ansonsten die Präzision und Empfindlichkeit der Methode zu stark variieren kann. Darüber hinaus ist die Abfüllung von Suspensionen der inerten Partikel im Mikroliterbereich technisch sehr schwierig, insbesondere weil das ständige erforderliche Rühren während der Abfüllung die inerten Partikel beschädigen und man damit wiederum das Volumenergebnis verfälschen kann. Noch ein weiterer Nachteil des bekannten Verfahrens besteht darin, daß es im wesentlichen auf farbige trägergebundene Antigene oder Antikörper, wie z.B. Erythrozyten, limittiert ist. Weiße bzw. farblose Reagenzien, wie sie z.B. bei Leukozyten-, Thrombozyten- oder Latexreaktionen vorliegen, können ohne vorherige Einfärbung nicht erfaßt werden.

Eine der vorliegenden Erfindung zugrunde liegende Aufgabe bestand somit darin, die obengenannten Nachteile, die sich durch die Verwendung einer aus inerten Partikeln bestehenden Matrix ergeben, mindestens teilweise zu beseitigen.

Diese Aufgabe wird gelöst durch ein Verfahren zum Nachweis eines Analyten in einer Probeflüssigkeit durch Agglutination, wobei man die Probeflüssigkeit mit einem Agglutinationsreagenz in Kontakt bringt und eine Reaktion zwischen dem Analyten und dem Agglutinationsreagenz bestimmt, welches dadurch gekennzeichnet ist, daß man ein Reaktionsgefäß verwendet, das eine kompakte poröse Matrix enthält und das nach Einwirkung von Gravitationskräften eine qualitative oder semiquantitative Bestimmung der Agglutinationsreaktion erlaubt.

Überraschenderweise wurde festgestellt, daß alle scheinbar unlösbaren, bei der Verwendung inerter Partikel auftretender Probleme überwunden werden können, indem anstelle der einzelnen inerten Partikel eine einzige kompakte Matrix verwendet wird.

Vorzugsweise verwendet man für das erfindungsgemäße Verfahren eine kompakte Matrix, die Kanäle, insbesondere Kanäle mit definierten Durchmesser, enthält. Der Innendurchmesser der Poren bzw. Kanäle der Matrix kann entsprechend dem jeweiligen Test stark variieren. So werden für kleine Thrombozyten Kanäle mit geringerem Durchmesser als für die relativ großen Leukozyten verwendet.

Die kompakten Matrizes sind mit verschiedenen Porenweiten erhältlich, wie im folgenden angegeben ist.

| Kennzeichnung | Kennzeichnung ISO 4793 | Nennwerte der Porenweite µm |
|---|---|---|
| G0 | P250 | 160-250 |
| G1 | P160 | 100-160 |
| G2 | P100 | 40-100 |
| G3 | P 40 | 16-40 |
| G4 | P 16 | 10-16 |
| G5 | P 1,6 | 1,0-1,6 |

Vorzugsweise verwendet man eine kompakte Matrix auf Basis von Glas oder Kunststoff. Besonders bevorzugt ist eine Glasmatrix, z.B. eine Matrix aus Controlled Pore Glass. Derartige Matrizes sind z.B. aus Duran. Diese sind von der Firma Brand, Deutschland, mit verschiedenen Kanaldurchmessern erhältlich. Um mögliche unspezifische Absorptionen zu vermeiden, wird das Glas vorzugsweise mit einer modifizierten, z.B. silanisierten Oberfläche eingesetzt.

Das erfindungsgemäße Verfahren betrifft den Nachweis eines Analyten in einer Probeflüssigkeit. Als Probeflüssigkeit dienen vorzugsweise Körperflüssigkeiten, die gegebenenfalls verdünnt sein können, z.B. Blut, Serum oder Plasma. Das Volumen der Probeflüssigkeit für das erfindungsgemäße Verfahren kann in weiten Bereichen variieren, vorzugsweise werden für Mikrotests Volumina von 1 bis 200 µl verwendet.

Zum Nachweis des Analyten wird ein Agglutinationsreagenz verwendet, d.h. eine spezifisch und mit hoher Affinität mit dem Analyten bindefähige Substanz. Das Agglutinationsreagenz enthält mindestens zwei Bindungsstellen für den Analyten, wodurch das Entstehen von vernetzten Agglutinationskomplexen aus Analyt und Agglutinationsreagenz ermöglicht wird. Im Falle, daß ein immobilisierter, z.B. an die Matrix gebundener Antikörper eingesetzt wird, genügt natürlich eine einzige Bindungsstelle.

Die durch das erfindungsgemäße Verfahren nachweisbaren Analyten sind Substanzen, die eine spezifische und hochaffine Wechselwirkung mit dem Agglutinationsreagenz eingehen können, z.B. Antigene bzw. Antikörper, die durch eine Immunreaktion bestimmt werden können. Eine erste bevorzugte Ausführungsform der vorliegenden Erfindung betrifft den Nachweis von Antikörpern als Analyten in der Probeflüssigkeit, z.B. Antikörper gegen Pathogene, wie etwa Viren (HIV, Hepatitis-Viren), Bakterien oder Protozoen, Antikörper gegen Autoantigene, Antikörper gegen Tumoren oder Antikörper gegen Allergene. Die Antikörper können freie Antikörper, z.B. IgG, oder zellständige Antikörper, z.B. IgE, sein. Zum Nachweis von freien Antikörpern, d.h. insbesondere nicht-zellgebundenen Antikörpern, verwendet man zweckmäßigerweise ein trägergebundenes Antigen als Agglutinationsreagenz. Beispiele für geeignete Träger sind synthetische Träger, wie etwa Partikel aus Latex, Dextrose, Agarose, vernetzten Polypeptiden etc. und natürliche Träger, wie etwa Zellen, z.B. Erythrozyten. Vorzugsweise verwendet man einen markierten, d.h. mit einer nachweisbaren Gruppe versehenen Träger. Bei Verwendung von gefärbten Trägern, wie etwa Erythrozyten, ist eine zusätzliche Einführung von Markierungsgruppen nicht erforderlich. Weiterhin kann bei bestimmten Ausführungsformen der vorliegenden Erfindung, wie im folgenden näher erläutert, völlig auf die Einführung von Markierungsgruppen verzichtet werden.

Eine weitere bevorzugte Ausführungsform der vorliegenden Erfindung betrifft den Nachweis von Antigenen in einer Probeflüssigkeit, z.B. freie Antigene, wie etwa Serumproteine, Metaboliten, Hormone, Mediatoren etc. oder trägergebundene Antigene, wie etwa zelluläre Blutgruppen-Antigene etc. Zum Nachweis trägergebundener Antigene verwendet man vorzugsweise freie Antikörper oder bivalente Fragmente davon als Agglutinationsreagenz. Zum Nachweis freier Antigene verwendet man vorzugsweise trägergebundene Antikörper bzw. trägergebundene Antikörperfragmente.

Beim erfindungsgemäßen Verfahren wird ein Reaktionsgefäß mit einer kompakten porösen Matrix verwendet, das nach Einwirkung von Gravitationskräften eine qualitative oder semiquantitative Bestimmung der Agglutinationsreaktion zwischen dem zu bestimmenden Analyten und dem Agglutinationsreagenz erlaubt. Obwohl die Einwirkung von Gravitationskräften auch durch längere Sedimentation verursacht werden kann, verwendet man vorteilhafterweise eine Zentrifugation, da schon nach kurzer Zeit die gewünschte Sedimentation bewirkt werden kann. Die optimalen Bedingungen hinsichtlich Zentrifugationsdauer und g-Zahl kann der Fachmann ohne Schwierigkeiten für jedes Analysesystem ermitteln. Diese Bedingungen werden insbesondere durch die Beschaffenheit des Agglutinationskomplexes zwischen Agglutinationsreagenz und Analyt, der Komponenten des Reaktionsgemisches in ungebundenem Zustand sowie der Porengröße bzw. der Porengrößenverteilung der kompakten Matrix bestimmt.

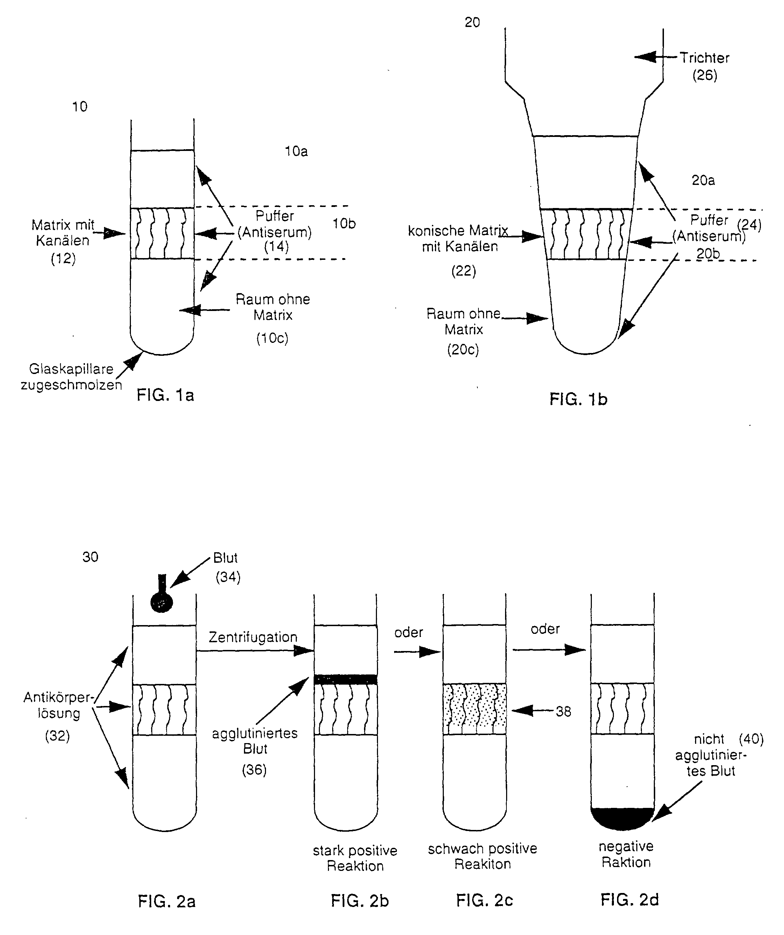

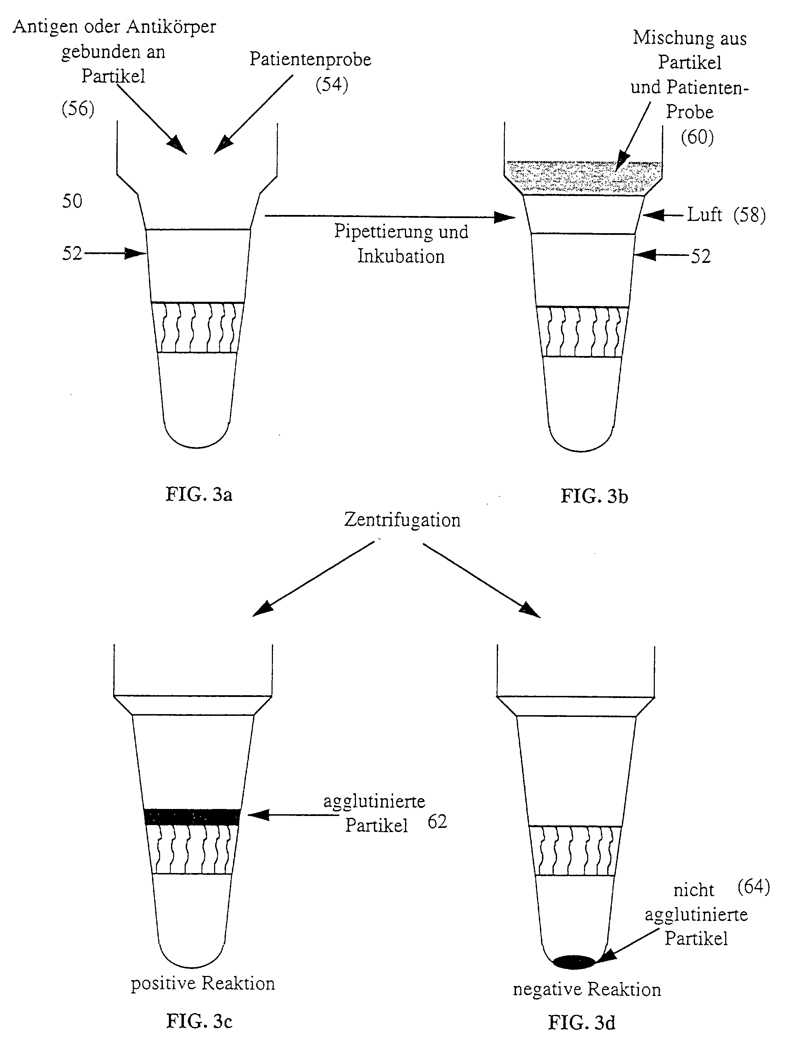

Vorzugsweise wird die Porengröße der Matrix so ausgewählt, daß bei einer starken Agglutination das Reaktionsprodukt aus dem zu bestimmenden Analyten und dem Agglutinationsreagenz im wesentlichen nicht in die kompakte Matrix eindringen kann. Dieses Reaktionsmuster ist z.B. in Fig. 2b und Fig. 3c erläutert.

Bei einer schwach positiven Reaktion zwischen Analyt und Agglutinationsreagenz soll das Reaktionsprodukt vorzugsweise in die kompakte Matrix zwar eindringen, sie aber nicht vollständig durchdringen. Dieses Reaktionsmuster ist in Fig. 2c erläutert. Bei Fehlen einer signifikanten Agglutinationsreaktion zwischen Agglutinationsreagenz und zu bestimmenden Analyten können im Reaktionsgefäß enthaltene Komponenten, insbesondere trägergebundene Antikörper, Antigene bzw. ein trägergebundenes Agglutinationsreagenz die kompakte Matrix im wesentlichen vollständig durchdringen. Ein dabei resultierendes Reaktionsmuster ist in Fig. 2d und Fig. 3d dargestellt. Bei Verwendung eines Reaktionsgefäßes, bei dem die Matrix nicht bis zum Gefäßboden reicht, ist bei negativen Reaktionen auch die Erfassung von nicht gefärbten Trägern möglich, da das Sediment durch eine Flüssigkeitsschicht von der Matrix abgegrenzt ist. Bei einer schwach positiven Reaktion läßt die Höhe des Sediments eine semiquantitative Messung zu.

Die Vermischung von Agglutinationsreagenz und Analyt kann in bestimmten Ausführungsformen des erfindungsgemäßen Verfahrens erfolgen, während beide Komponenten in Kontakt mit der Matrix sind. In anderen Ausführungsformen ist jedoch eine Vorinkubation erforderlich. Diese Vorinkubation kann in einem separaten Gefäß erfolgen. Vorzugsweise erfolgt die Vorinkubation jedoch innerhalb des Reaktionsgefäßes, das auch die Matrix enthält. In diesem Fall muß das Reaktionsgefäß so ausgebildet sein, daß kein sofortiger Kontakt von in das Gefäß pipettierten Lösungen mit der Matrix stattfindet. Dies kann beispielsweise durch Anbringen einer Membran oberhalb der Matrix oder - wie im folgenden ausführlich erläutert - durch Verwendung von speziell ausgeformten Reaktionsgefäßen erreicht werden.

Weiterhin soll die vorliegende Erfindung durch die nachfolgenden Figuren erläutert werden. Es zeigen:
- Fig. 1a: eine erste Ausführungsform eines Reaktionsgefäßes im Querschnitt mit kompakter Matrix und Kanälen, beispielsweise eine im Handel erhältliche Glaskapillare, die eine integrierte Matrix enthält;
- Fig. 1b: eine zweite Ausführungsform eines Reaktionsgefäßes im Querschnitt mit kompakter Matrix und Kanälen, beispielsweise Mikroreaktionsgefäße, wie sie im Handel z.B. von DiaMed und Ortho erhältlich sind und die eine integrierte Matrix enthalten;
- Fig. 2: die Reaktion eines trägergebundenen Antigens in einer Probeflüssigkeit mit einem Agglutinationsreagenz in einem Reaktionsgefäß gemäß Fig. 1a;
- Fig. 3: die Reaktion eines trägergebundenen Agglutinationsreagenz mit in einer Probeflüssigkeit vorhandenen spezifischen Antikörpern in einem Reaktionsgefäß gemäß Fig. 1b;
- Fig. 4: eine dritte Ausführungsform eines Reaktionsgefäßes in der Ansicht von oben, wobei das Röhrchen für Testausführungen in horizontaler Lage konzipiert ist, durch die Verwendung einer kompakten Matrix kann sich die Oberfläche der Matrix nicht verformen;
- Fig. 5: eine mögliche Anordnung der Reagenzgefäße gemäß Fig. 4 auf einer runden Scheibe;
- Fig. 6: ein Fließdiagramm, mit dem die Bestimmung von Analyten unter Verwendung der Anordnung gemäß Fig. 5 einfach automatisiert werden kann.

In Fig. 1a und 1b sind zwei bevorzugte Ausführungsformen für erfindungsgemäße Reaktionsgefäße gezeigt. Diese Reaktionsgefäße (10, 20) enthalten einen oberen Bereich (10a, 20a), einen mittleren Bereich (10b, 20b) und einen unteren Bereich (10c, 20c), wobei sich eine kompakte poröse Matrix (12, 22) über den mittleren Bereich (10b 20b) erstreckt und die oberen (10a, 20a) bzw. unteren (10c, 20c) Bereiche einen Raum ohne Matrix umfassen. Im Reaktionsgefäß befindet sich weiterhin eine Flüssigkeit (14, 24), z.B. eine Puffer- oder Reagenzlösung, deren Flüssigkeitsspiegel sich oberhalb des oberen Endes der Matrix befindet. Das in Fig. 1b gezeigte Reaktionsgefäß (20) enthält außerdem im oberen Bereich (20a) eine Erweiterung (26), z.B. in Form eines Trichters.

Als Reaktionsgefäße werden vorzugsweise Mikrozentrifugationsröhrchen mit einem Volumen von 50 µl bis 2 ml, insbesondere von 50 µl bis 1 ml, verwendet. Bei einem Mikrozentrifugationsgefäß (20) mit Erweiterung (26) liegt der Flüssigkeitsspiegel vor Zugabe des Agglutinationsreagenz zu der Probe vorzugsweise unterhalb der Erweiterung (26), so daß bei Pipettierung der Probe und des Agglutinationsreagenz sich eine Luftblase ausbilden kann, die einen sofortigen Kontakt der zupipettierten Flüssigkeiten mit der Matrix verhindert.

Das Reaktionsröhrchen kann jeweils entsprechend der Ausführungsform nach unterschiedlichen Verfahren mit der Matrix bestückt und mit Reagenz (Puffer oder Antiserum) gefüllt werden. Dabei empfiehlt sich folgendes Vorgehen: Aus einer handelsüblichen Glasfritte wird ein Stück in der für die Öffnung passende Form herausgetrennt und in die Kapillare gesetzt. Diese kann durch einfaches Erwärmen mit der Matrix verbunden werden. Die Füllung erfolgt durch einfache Kapillarwirkung, indem die Kapillare in das Reagenz getaucht wird. Dadurch wird die Flüssigkeit eingezogen und die Luft von unten herausgedrückt. Der Prozeß kann je nach gewünschter Füllhöhe jederzeit abgebrochen werden. Das in Fig. 1a dargestellte Reaktionsröhrchen wird durch einfaches Abschmelzen erhalten. Gegebenenfalls, wenn eine längere Lagerung vorgesehen ist, kann auch die obere Öffnung der Kapillare zugeschmolzen werden. Zur Anwendung wird der obere Teil einfach abgebrochen, gegebenenfalls mit Hilfe einer Bruchrille. Das hier beschriebene manuelle Herstellungsverfahren kann natürlich mit heutzutage zur Verfügung stehenden Hilfsmitteln sehr kostengünstig maschinell in großer Stückzahl erfolgen.

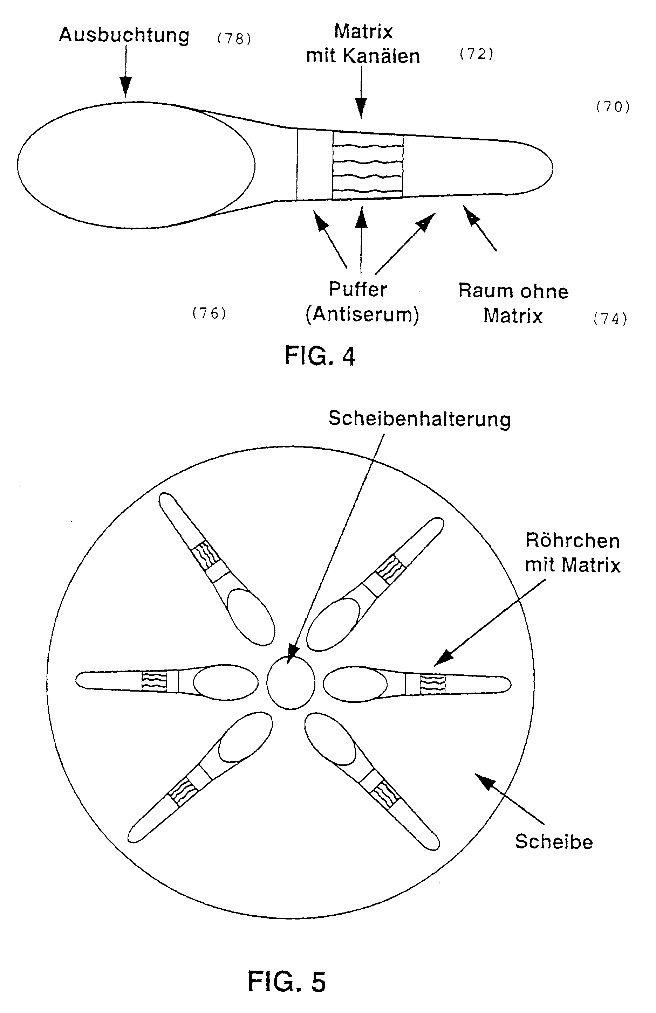

Für die Füllung der Reaktionsgefäße gemäß Fig. 1b empfiehlt sich folgendes Vorgehen: In das leicht konisch geformte Gefäß wird das entsprechende Reagenz eingefüllt und anschließend die ebenfalls leicht konisch geformte Matrix zugefügt. Matrix und Gefäßwand passen sich aufgrund der Schwerkraft genau einander an. Diese Anpassung kann natürlich durch Zentrifugation beschleunigt und verfestigt werden. Für Reaktionsgefäße gemäß Fig. 4, die für eine horizontale Anwendung vorgesehen sind, empfiehlt sich eine dritte Herstellungsmethode: Die Matrix wird vor der Reagenzzugabe in das Röhrchen eingepaßt. Das Reagenz wird in die Ausbuchtung des Röhrchens gegeben und durch Zentrifugation in den unteren Teil transferiert, dadurch wird gleichzeitig die Matrix an die Röhrchenwand angedrückt, während die im Röhrchen vorhandene Luft herausgepreßt wird. Eine industrielle Herstellung ist auch nach diesem Verfahren leicht realisierbar.

An die Oberfläche der Matrix können für bestimmte Ausführungsformen des erfindungsgemäßen Verfahrens auch Antigene bzw. Antikörper immobilisiert werden.

Fig. 2 zeigt ein Reaktionsschema zum Nachweis eines trägergebundenen Antigens in einer Probeflüssigkeit, z.B. die Bestimmung eines Blutgruppenmerkmals in einer Patientenprobe unter Verwendung des in Fig. 1a dargestellten Reaktionsgefäßes und Antikörpern als Agglutinationsreagenz.

Hierzu wird in ein Reaktionsgefäß (30) mit einer kompakten Matrix, in dem sich eine, Antikörper gegen den zu bestimmenden Analyten enthaltende Lösung (32) befindet, eine Probeflüssigkeit (34) z.B. Blut, gegeben, die einen trägergebundenen Analyten, z.B. ein an der Oberfläche von Erythrozyten gebundenes Blutgruppenmerkmal, enthält (Fig. 2a). Nach Zentrifugation findet man bei einer stark positiven Agglutinationsreaktion (Fig. 2b) eine Bande von Agglutinationsprodukten (36) auf der Oberseite der Matrix. Wenn nur eine schwach positive Reaktion stattfindet, findet man das Agglutinationsprodukt (38) innerhalb der kompakten Matrix (vgl. Fig. 2c). Bei einer negativen Reaktion, d.h. wenn kein Analyt in der Probe anwesend ist, so daß keine Reaktion mit dem Agglutinationsreagenz stattfinden kann, lagern sich die nicht-agglutinierten trägergebundenen Komponenten des Reaktionsgemisches (40) aufgrund der Gravitationskraft am Boden des Reaktionsgefäßes ab (vgl. Fig. 2d).

Fig. 3 zeigt ein Reaktionsschema für den Nachweis von in der Probeflüssigkeit vorhandenen spezifischen Antikörpern unter Verwendung eines trägergebundenen Antigens als Agglutinationsreagenz in einem Reaktionsgefäß gemäß Fig. 1b. Hierzu werden in ein Reaktionsgefäß (50), das eine geeignete Flüssigkeit (52), z.B. eine Pufferlösung oder einen zweiten Antikörper (Coombs-Test), enthält, eine Probeflüssigkeit (54) und ein trägergebundenes Antigen, z.B. ein an der Oberfläche von Latexpartikeln fixiertes Antigen, (56) gegeben. Vorzugsweise erfolgt die Zugabe derart, daß die Probeflüssigkeit und das trägergebundene Antigen eine durch eine Luftblase (58) von der Flüssigkeit (52) getrennte Mischung (60) ergeben (vgl. Fig. 3b). Nach einer geeigneten Inkubationsdauer wird die Mischung (60), z.B. durch Zentrifugation, in Kontakt mit der Matrix gebracht. Bei einer positiven Reaktion bildet sich ein Agglutinationsprodukt (62) an der Oberseite der Matrix (vgl. Fig. 3c), während bei einer negativen Reaktion das nicht-agglutivierte Agglutinationsreagenz, d.h. das trägergebundene Antigen (64), die Kanäle der Matrix durchdringt und am Boden des Reaktionsgefäßes sedimentiert (vgl. Fig. 3d).

Vorzugsweise werden zur Durchführung des erfindungsgemäßen Verfahrens Mikroreaktionsgefäße verwendet, die auf einer Karte oder Scheibe in beliebiger Anzahl nebeneinander angeordnet werden können. Eine solche Testkarte kann auf verschiedene Arten hergestellt sein. Beispielsweise können Röhrchen auf eine Karte oder Scheibe geklebt sein oder aber die Röhrchen können in die Karte oder Scheibe integriert ausgebildet sein.

Dabei können die Reaktionsgefäße bereits ein Agglutinationsreagenz enthalten und mit einer aufgeschweißten Folie verschlossen sein. Auf diese Weise hergestellte.Test ermöglichen eine einfache Handhabung und können in einem automatisierten Analyseverfahren eingesetzt werden. Dabei kann die Probenzugabe in die einzelnen Röhrchen, die Probenbehandlung und die Auswertung mittels elektronischer Datenverarbeitung gesteuert werden.

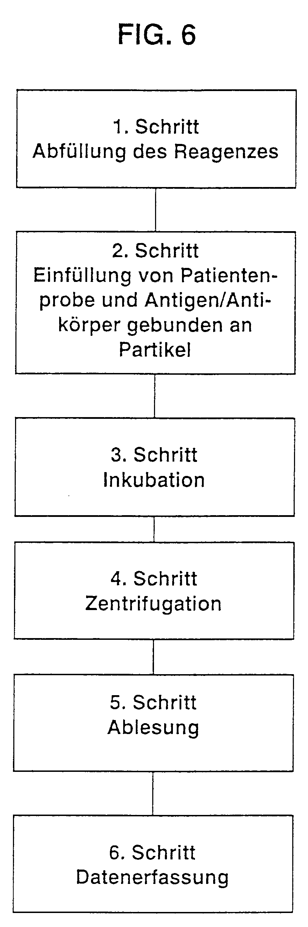

Die kompakte Matrix erlaubt ferner ein überraschendes Verfahren, mit welchen Agglutinationsreaktionen besonders einfach und kostengünstig automatisiert werden können. Dazu können Reaktionsgefäße (70) gemäß Fig. 4 verwendet werden. Solche Reaktionsgefäße enthalten eine Matrix mit Kanälen (72) sowie einen Raum ohne Matrix (74) und sind mit einer geeigneten Flüssigkeit (76), z.B. Puffer oder Antiserum, bis über das obere Matrixende gefüllt. Weiterhin weisen die Reaktionsgefäße eine Erweiterung (78), z.B. in Form einer Ausbuchtung aus. Die Reaktionsgefäße sind vorzugsweise horizontal auf einer Scheibe (Fig. 5) in beliebiger Anzahl angebracht oder integriert. Die dadurch bis heute nicht erreichte Einfachheit der Reaktionsschritte wird im Fließdiagramm gemäß Fig. 6 erläutert: Im ersten Schritt wird das für die jeweilige Reaktion benötigte Reagenz, sei es Puffer oder Antiserum, in die Ausbuchtung des Röhrchens pipettiert. Durch Zentrifugation wird die Flüssigkeit in den unteren Teil des Röhrchens überführt, wobei gleichzeitig die im Röhrchen vorhandene Luft herausgedrückt wird. Dieser erste Schritt kann entweder unmittelbar vor der eigentlichen Analyse oder vorher in einem industriellen Fertigungsprozeß erfolgen. Im letzteren Fall müssen die Reaktionsgefäße selbstverständlich dicht verschlossen werden, beispielsweise durch Verschweißung mit einer Folie. Im 2. Schritt wird die Patientenprobe und bei Reaktionen gemäß Fig. 3 Antigen gebunden an Partikel in die Ausbuchtung gegeben. Im 3. Schritt reagieren Antigene und Antikörper in der Inkubationsphase. Dieser Schritt kann natürlich bei Reaktionen gemäß Fig. 2 übersprungen werden. Im 4. Schritt wird zentrifugiert, wobei sich die Reaktionsmuster gemäß Fig. 2b, 2c, 2d ausbilden. Diese Reaktionsmuster werden im 5. Schritt automatisch gelesen (z.B. durch einen Scanner) und in einem Computerprogramm interpretiert. Im 6. Schritt werden die Daten zur Erfassung übertragen, wobei jedes Ergebnis zur Sicherheit visuell verifiziert werden kann.

Noch ein weiterer Gegenstand der vorliegenden Erfindung ist ein Reagenzienkit zum Nachweis eines Analyten in einer Probeflüssigkeit durch Agglutination, umfassend
(a) ein Reaktionsgefäß, das eine kompakte poröse Matrix enthält und
(b) ein zur Bildung von Agglutinationskomplexen mit dem Analyten fähiges Agglutinationsreagenz.

Das Reaktionsgefäß und das Agglutinationsreagenz können in physikalisch getrennter Form vorliegen. Andererseits kann bei bestimmten Testformaten das Agglutinationsreagenz bereits im Reaktionsgefäß vorliegen. Die kompakte Matrix ist vorzugsweise in das Reaktionsgefäß derart eingefügt, daß bei einer Zentrifugation keine Verschiebungen stattfinden können. Weiterhin steht die Matrix zweckmäßigerweise im direkten Kontakt mit der Innenseite des Reaktionsgefäßes, so daß bei einer Zentrifugation keine Komponenten des Reaktionsgemisches zwischen Reaktionsgefäß-Innenwand und Matrix hindurchtreten können. Die Matrix erstreckt sich günstigerweise nicht über den gesamten Bereich des Reaktionsgefäßes, sondern nur über einen mittleren Bereich.

In einer bevorzugten Ausführungsform der vorliegenden Erfindung enthält das Reagenzienkit mehrere Reaktionsgefäße, die zusammen auf einer Karte oder Scheibe angeordnet sind. Die auf einer Karte oder Scheibe befindlichen Reaktionsgefäße können zum Nachweis desselben Analyten oder zum Nachweis unterschiedlicher Analyten vorgesehen sein. Das Volumen der Reaktionsgefäße ist vorzugsweise von 50 µl bis 2 ml.

Noch ein weiterer Gegenstand der vorliegenden Erfindung ist ein Reaktionsgefäß, das eine kompakte poröse Matrix enthält, wobei die Matrix derart angeordnet ist, daß bei Zentrifugation des Gefäßes keine Verschiebung der Matrix innerhalb des Gefäßes stattfindet. Vorzugsweise ist das Reaktionsgefäß mindestens teilweise optisch transparent, so daß eine einfache Bestimmung des Auftretens bzw. Fehlens einer Agglutinationsreaktion möglich ist.

Die Erfindung wird durch die nachfolgenden Beispiele näher erläutert.

### Beispiel 1: Herstellung der Reaktionsgefäße

a) Aus Frittenmaterial (Duran, Firma Brand, Deutschland) wird die kompakte Matrix entsprechend der gewünschten Form (Fig. 1a oder 1b) zugeschnitten.
b) Die kompakte Matrix wird in Königswasser (1 Teil HNO₃ und 3 Teile HCl) 5 min. zur Aktivierung aufgekocht, mit destilliertem Wasser gut ausgewaschen, getrocknet und anschließend mit Tetramethoxysilan, Fluka (0,001 % in trockenem Methanol) 30 min. bei Raumtemperatur silanisiert, mit destilliertem Wasser gewaschen und getrocknet.
c) Die silanisierte Matrix wird anschließend für Reaktionen in Glaskapillaren (Fig. 1a) in diese gegeben und durch kurzes Erhitzen mit der Glaswand der Kapillare verbunden. Für Reaktionsgefäße gemäß Fig. 1b mit konisch geformter Matrix ist dieser Schritt nicht erforderlich, da die Matrix aufgrund ihrer Form automatisch in der gewünschten Position im Reaktionsgefäß fixiert wird.

### Beispiel 2: Füllung der Reaktionsgefäße

a) Eine Glaskapillare gemäß Fig. 1a wird in die gewünschte Puffer- oder Antiserenlösung getaucht, durch die Kapillarkräfte füllt sich die Kapillare automatisch bis zur gewünschten Füllhöhe, vorzugsweise kurz über der kompakten Matrix. Der Boden der Kapillare wird nach der Füllung zugeschmolzen oder durch eine Kitmasse verschlossen. Das obere Ende der Kapillare kann ebenfalls so dicht verschlossen werden. Zum Gebrauch wird die obere Öffnung der Kapillare z.B. durch eine von Ampullen her bekannte Bruchrille versehen.
b) Bei Reaktionsgefäßen gemäß Fig. 1b wird der Puffer oder das Antiserum zuerst in die Reaktionsgefäße gefüllt und die kompakte Matrix erst nachträglich zugegeben. Die Reaktionsgefäße können nach der Füllung problemlos nach bekannten Verfahren verschlossen werden.
c) Bei Reaktionsgefäßen gemäß Fig. 4 und 5 wird der Puffer oder das Antiserum in die Ausbuchtung gegeben und durch kurzes Zentrifugieren (5 min., 900 rpm) in den unteren Teil des Reaktionsröhrchens übergeführt.

### Beispiel 3: Nachweis der Blutgruppe A

a) Nachweis in einer Kapillare gemäß Fig. 1a
   Eine Glaskapillare (100 µl) wird gemäß Beispiel 1 mit einer kompakten Matrix (Gl) versehen und gemäß Beispiel 2 in ein kommerziell erhältliches Anti-A-Reagenz (DiaMed) getaucht. Sobald der Flüssigkeitsspiegel über der Matrix ist, wird die Kapillare herausgezogen und unten durch kurzes Erwärmen zugeschmolzen. Anschließend werden 50 µl einer 5 %-Suspension (0,9 % NaCl) des Patientenblutes zugegeben und in einer Heraeus-Zentrifuge bei 1000 rpm 6 Minuten zentrifugiert. Bei einer positiven Reaktion liegen die agglutinierten Erythrozyten auf der Matrix (Fig. 2b), bei einer negativen Reaktion liegen die freien Erythrozyten auf dem Boden der Kapillare (Fig. 2d).
b) Nachweis in Reaktionsgefäßen gemäß Fig 1b
   Die Reaktionsgefäße (DiaMed) werden wie im Beispiel 1 hergestellt und gemäß Beispiel 2b mit 25 µl Anti-A gefüllt. Anschließend werden 20 µl einer 5 %-Suspension (0,9 % NaCl) des Patientenblutes zugegeben und 10 min. bei 910 rpm zentrifugiert (Zentrifuge DiaMed). Die Reaktionsmuster sind gleich wie im Beispiel 3a.
c) Nachweis auf einer Scheibe gemäß Fig. 5
   Die Reaktionsgefäße (Fig. 4) werden, wie in den Beispielen 1 und 2c beschrieben, hergestellt und mit 25 µl Anti-A gefüllt. Anschließend wird in die Ausbuchtung 20 µl einer 5 %-Suspension des Patientenblutes gegeben und die Scheibe 10 min. bei 910 rpm in einer für Scheiben modifizierte Zentrifuge (DiaMed) zentrifugiert. Die Reaktionsmuster sind gleich wie in den Beispielen 3a und 3b).

### Beispiel 4: Nachweis von Antikörpern, die gegen Antigene auf Erythrozyten gerichtet sind

Die Reaktionsgefäße werden wie zuvor beschrieben hergestellt und mit einer Antikörperlösung gefüllt. Nur wird in diesem Falle anstelle des Anti-A, ein Coombs-Serum (Antihuman IgG) eingefüllt. Geeignete gefüllte Gefäße, die einen Trichter (Fig. 1b) oder eine Ausbuchtung (Fig. 4) enthalten, werden mit 50 µl einer 0,8 %-Erythrozyten-Suspension (Testzellen DiaMed) gefüllt und mit 25 µl Patientenserum gemischt. In dem Trichter (Fig. 1b) oder der Ausbuchtung (Fig. 4) wird die Mischung aus Testzellen und Serum 15 min. bei 37 °C inkubiert. Nach Zentrifugation sind die Reaktionsmuster gleich wie in den vorhergehenden Beispielen. Bei einer schwachen Antikörperreaktion bildet sich ein Reaktionsmuster gemäß Fig. 2c.

## Patentansprüche

1. Verfahren zum Nachweis eines Analyten in einer Probeflüssigkeit durch Agglutination, wobei man die Probeflüssigkeit mit einem Agglutinationsreagenz in Kontakt bringt und eine Reaktion zwischen dem Analyten und dem Agglutinationsreagenz bestimmt,
**dadurch gekennzeichnet,**
**daß** man ein Reaktionsgefäß verwendet, das eine einzige kompakte poröse Matrix enthält und das nach Einwirkung von Gravitationskräften eine qualitative oder semiquantitative Bestimmung der Agglutinationsreaktion erlaubt.

2. Verfahren nach Anspruch 1,
**dadurch gekennzeichnet,**
**daß** man eine kompakte Matrix verwendet, die Kanäle mit definiertem Durchmesser aufweist.

3. Verfahren nach Anspruch 1 oder 2,
**dadurch gekennzeichnet,**
**daß** man eine Matrix auf Basis von Glas oder Kunststoff verwendet.

4. Verfahren nach Anspruch 3,
**dadurch gekennzeichnet,**
**daß** man eine oberflächenmodifizierte Glasmatrix verwendet.

5. Verfahren nach Anspruch 3 oder 4,
**dadurch gekennzeichnet,**
**daß** man eine silanisierte Glasmatrix verwendet.

6. Verfahren nach einem der Ansprüche 1-5,
**dadurch gekennzeichnet,**
**daß** man die Porengröße der Matrix so auswählt, so daß nach Einwirkung von Gravitationskräften
(a) bei einer starken Agglutination das Reaktionsprodukt aus dem zu bestimmenden Analyten und dem Agglutinationsreagenz im wesentlichen nicht in die kompakte Matrix eindringen kann,
(b) bei einer schwachen Agglutination das Reaktionsprodukt in die kompakte Matrix eindringt, sie aber nicht vollständig durchdringen kann und
(c) beim Fehlen einer Agglutination die im Reaktionsgefäß enthaltenen Komponenten die kompakte Matrix im wesentlichen vollständig durchdringen können.

7. Verfahren nach einem der Ansprüche 1-6,
**dadurch gekennzeichnet,**
**daß** man zum Nachweis eines freien Antikörpers ein trägergebundenes Antigen als Agglutinationsreagenz verwendet.

8. Verfahren nach einem der Ansprüche 1-6,
**dadurch gekennzeichnet,**
**daß** man zum Nachweis eines trägergebundenen Antigens einen freien Antikörper oder ein Fragment davon als Agglutinationsreagenz verwendet.

9. Verfahren nach einem der Ansprüche 1-6,
**dadurch gekennzeichnet,**
**daß** man zum Nachweis eines freien Antigens einen trägergebundenen Antikörper bzw. ein trägergebundenes Antikörperfragment verwendet.

10. Verfahren nach einem der Ansprüche 1-9,
**dadurch gekennzeichnet,**
**daß** Agglutinationsreagenz und Probeflüssigkeit vorgemischt werden, bevor sie mit der Matrix in Kontakt gebracht werden.

11. Verfahren nach einem der Ansprüche 1-10,
**dadurch gekennzeichnet,**
**daß** man ein Reaktionsgefäß (10, 20) mit einem oberen Bereich (10a, 20a), einem mittleren Bereich (10b, 20b) und einem unteren Bereich (10c, 20c) verwendet, wobei sich die Matrix (12, 22) über den mittleren Bereich (10b, 20b) erstreckt und die oberen (10a, 20a) bzw. unteren (10c, 20c) Bereiche einen Raum ohne Matrix umfassen.

12. Verfahren nach Anspruch 11,
**dadurch gekennzeichnet,**
**daß** der obere Bereich (20a) des Reaktionsgefäßes (20) eine Erweiterung (26, 78) enthält.

13. Verfahren nach Anspruch 12,
**dadurch gekennzeichnet,**
**daß** die Erweiterung in Form eines Trichters oder einer Ausbuchtung ist.

14. Verfahren nach einem der Ansprüche 1-13,
**dadurch gekennzeichnet,**
**daß** man als Reaktionsgefäße Mikrozentrifugationsröhrchen mit einem Volumen von 50 µl bis 2 ml verwendet.

15. Reagenzienkit zum Nachweis eines Analyten in einer Probeflüssigkeit durch Agglutination, umfassend
(a) ein Reaktionsgefäß, das eine einzige kompakte poröse Matrix enthält, und
(b) ein zur Bildung von Agglutinationskomplexen mit dem Analyten fähiges Agglutinationsreagenz.

16. Reagenzienkit nach Anspruch 15,
**dadurch gekennzeichnet,**
**daß** das Reaktionsgefäß (10, 20) einen oberen Bereich (10a, 20a), einen mittleren Bereich (10b, 20b) und einen unteren Bereich (10c, 20c) umfaßt, wobei sich die Matrix (12, 22) nur über den mittleren Bereich (10b, 20b) erstreckt.

17. Reagenzienkit nach Anspruch 15 oder 16,
**dadurch gekennzeichnet,**
**daß** mehrere Reaktionsgefäße zusammen auf einer Karte oder Scheibe angeordnet sind.

18. Reagenzienkit nach einem der Ansprüche 15-16,
**dadurch gekennzeichnet,**
**daß** das Reaktionsgefäß ein Volumen von 50 µl bis 2 ml aufweist.

19. Reaktionsgefäß, das eine einzige kompakte poröse Matrix enthält, wobei die Matrix derart angeordnet ist, daß bei Zentrifugation keine Verschiebung der Matrix innerhalb des Gefäßes stattfindet und daß vorzugsweise keine Flüssigkeit zwischen Gefäßinnenwand und Matrix durchtreten kann.

20. Reaktionsgefäß nach Anspruch 19,
**dadurch gekennzeichnet,**
**daß** es einen oberen Bereich (10a, 20a), einen mittleren Bereich (10b, 20b) und einen unteren Bereich (10c, 20c) umfaßt, wobei sich die Matrix (12, 22) nur über den mittleren Bereich (10b, 20b) erstreckt.

21. Reaktionsgefäß nach Anspruch 19 oder 20,
**dadurch gekennzeichnet,**
**daß** der obere Bereich (20a) eine Erweiterung (26, 78), insbesondere in Form eines Trichters oder einer Ausbuchtung umfaßt.

22. Reaktionsgefäß nach einem der Ansprüche 19-21,
**dadurch gekennzeichnet,**
**daß** es ein Volumen von 50 µl bis 2 ml aufweist.

23. Anordnung von mehreren Reaktionsgefäßen zusammen auf einer Karte oder Scheibe,
**dadurch gekennzeichnet,**
**daß** die Reaktionsgefäße eine einzige kompakte poröse Matrix enthalten, wobei die Matrix derart angeordnet ist, daß bei Zentrifugation keine Verschiebung der Matrix innerhalb des Gefäßes stattfindet und daß vorzugsweise keine Flüssigkeit zwischen Gefäßinnenwand und Matrix durchtreten kann.

## Claims

1. A method of detecting an analyte in a test liquid by agglutination, wherein the test liquid is brought into contact with the agglutination reagent and a reaction between the analyte and the agglutination reagent is determined, **characterised in that** a reaction vessel is used which contains a single compact, porous matrix and which, following the action of gravitational forces, allows qualitative or semi-quantitative determination of the agglutination reaction.

2. A method according to Claim 1, **characterised in that** a compact matrix is used which exhibits channels of a defined diameter.

3. A method according to Claim 1 or 2, **characterised in that** a matrix is used based on glass or plastics material.

4. A method according to Claim 3, **characterised in that** a surface-modified glass matrix is used.

5. A method according to Claim 3 or 4, **characterised in that** a silanised glass matrix is used.

6. A method according to any one of Claims 1 to 5, **characterised in that** the pore size of the matrix is selected so that after the action of gravitational forces
(a) in the case of strong agglutination the reaction product of the analyte being assayed and the agglutination reagent cannot penetrate to any substantial degree into the compact matrix,
(b) in the case of weak agglutination the reaction product penetrates into the compact matrix but cannot pass through it completely.
(c) in the absence of any agglutination the components contained in the reaction vessel can pass through the compact matrix more or less completely.

7. A method according to any one of Claims 1 to 6, **characterised in that** for the detection of a free antibody use is made of a carrier-bound antigen as agglutination reagent.

8. A method according to any one of Claims 1 to 6, **characterised in that** for the detection of a carrier-bound antigen use is made of a free antibody or a fragment thereof as agglutination reagent.

9. A method according to any one of Claims 1 to 6, **characterised in that** for the detection of a free antigen use is made of a carrier-bound antibody or a carrier-bound antibody fragment.

10. A method according to any one of Claims 1 to 9, **characterised in that** the agglutination reagent and test liquid are premixed before being brought into contact with the matrix.

11. A method according to any one of Claims 1 to 10, **characterised in that** a reaction vessel (10,20) is used having an upper area (10a,20a), a middle area (10b,20b) and a lower area (10c,20c), wherein the matrix (12,22) extends over the middle area (10b,20b) and the upper (10a,20a) and lower (10c,20c) areas comprise a space without a matrix.

12. A method according to Claim 11, **characterised in that** the upper area (20a) of the reaction vessel (20) has a widened portion (26,78).

13. A method according to Claim 12, **characterised in that** the widened portion is in the form of a funnel or a bulge.

14. A method according to any one of Claims 1 to 13, **characterised in that** as reaction vessels use is made of microcentrifugation tubes with a volume of 50 µl to 2 ml.

15. A reagents kit for detecting an analyte in a test liquid by agglutination, comprising
(a) a reaction vessel which contains a compact, porous matrix and
(b) an agglutination reagent capable of forming agglutination complexes with the analyte.

16. A reagents kit according to Claim 15, **characterised in that** the reaction vessel (10,20) comprises an upper area (10a,20a), a middle area (10b,20b) and a lower area (10c,20c), wherein the matrix (12,22) extends only over the middle area (10b,20b).

17. A reagents kit according to Claim 15 or 16, **characterised in that** a plurality of reaction vessels are disposed jointly on a card or disk.

18. A reagents kit according to Claim 15 and Claim 16, **characterised in that** the reaction vessel has a volume of 50 µl to 2 ml.

19. A reaction vessel which contains a compact, porous matrix, the matrix being disposed so that on centrifugation there is no displacement of the matrix within the vessel and that preferably no liquid can pass between the inside wall of the vessel and the matrix.

20. A reaction vessel according to Claim 19, **characterised in that** it comprises an upper area (10a,20a), a middle area (10b,20b) and a lower area (10c,20c), wherein the matrix (12,22) extends only over the middle area (10b,20b).

21. A reaction vessel according to Claim 19 or 20, **characterised in that** the upper area (20a) comprises a widened portion (26,78), in particular in the form of a funnel or a bulge.

22. A reaction vessel according to any one of Claims 19 to 21, **characterised in that** it has a volume of 50 µl to 2 ml.

23. An arrangement of a plurality of reaction vessels together on a card or disc, **characterised in that** the reaction vessels contain a single compact, porous matrix, the matrix being disposed so that on centrifugation there is no displacement of the matrix within the vessel and that preferably no liquid can pass between the inside wall of the vessel and the matrix.

## Revendications

1. Procédé de mise en évidence d'un analyte dans un échantillon liquide par agglutination où l'on met en contact l'échantillon liquide avec un réactif d'agglutination et on détermine une réaction entre l'analyte et le réactif d'agglutination, **caractérisé en ce que** l'on utilise un récipient réactionnel qui contient une matrice poreuse compacte unique et qui permet une détermination qualitative ou semi-quantitative de la réaction d'agglutination après l'action de forces de gravitation.

2. Procédé selon la revendication 1 **caractérisé en ce que** l'on utilise une matrice compacte qui comporte des canaux de diamètre défini.

3. Procédé selon la revendication 1 ou 2
**caractérisé en ce que** l'on utilise une matrice à base de verre ou de matière plastique.

4. Procédé selon la revendication 3 **caractérisé en ce que** l'on utilise en matrice en verre modifiée en surface.

5. Procédé selon la revendication 3 ou 4
**caractérisé en ce que** l'on utilise une matrice en verre silanisée.

6. Procédé selon l'une des revendications 1 à 5 **caractérisé en ce que** l'on choisit la taille des pores de la matrice de manière qu'après l'action de forces de gravitation
(a) dans le cas d'une forte agglutination, le produit de la réaction de l'analyte à déterminer et du réactif d'agglutination ne puisse sensiblement pas pénétrer dans la matrice compacte,
(b) dans le cas d'une faible agglutination, le produit de la réaction pénètre dans la matrice compacte mais ne puisse pas la traverser totalement et
(c) en l'absence d'une agglutination, les composants contenus dans le récipient réactionnel puissent traverser la matrice compacte sensiblement totalement.

7. Procédé selon l'une des revendications 1 à 6 **caractérisé en ce que**, pour la mise en évidence d'un anticorps libre, on utilise un antigène lié à un support comme réactif d'agglutination.

8. Procédé selon l'une des revendications 1 à 6 **caractérisé en ce que**, pour la mise en évidence d'un antigène lié à un support, on utilise un anticorps libre ou un fragment de celui-ci comme réactif d'agglutination.

9. Procédé selon l'une des revendications 1 à 6 **caractérisé en ce que**, pour la mise en évidence d'un antigène libre, on utilise un anticorps lié à un support ou un fragment d'anticorps lié à un support.

10. Procédé selon l'une des revendications 1 à 9 **caractérisé en ce que** le réactif d'agglutination et l'échantillon liquide sont prémélangés avant d'être mis en contact avec la matrice.

11. Procédé selon l'une des revendications 1 à 10 **caractérisé en ce que** l'on utilise un récipient réactionnel (10, 20) comportant un domaine supérieur (10a, 20a), un domaine intermédiaire (10b, 20b) et un domaine inférieur (10c, 20c) où la matrice (12, 22) s'étend sur le domaine intermédiaire (10b, 20b) et les domaines supérieur (10a, 20a) et inférieur (10c, 20c) définissent un espace sans matrice.

12. Procédé selon la revendication 11 **caractérisé en ce que** le domaine supérieur (20a) du récipient réactionnel (20) contient un élargissement (26, 78).

13. Procédé selon la revendication 12 **caractérisé en ce que** l'élargissement est sous forme d'un entonnoir ou d'un bombement.

14. Procédé selon l'une des revendications 1 à 13 **caractérisé en ce que** l'on utilise des petits tubes de microcentrifugation d'un volume de 50 µl à 2 ml comme récipients réactionnels.

15. Kit de réactifs pour la mise en évidence d'un analyte dans un échantillon liquide par agglutination comprenant
(a) un récipient réactionnel qui contient une matrice poreuse compacte unique et
(b) un réactif d'agglutination capable de former des complexes d'agglutination avec l'analyte.

16. Kit de réactifs selon la revendication 15 **caractérisé en ce que** le récipient réactionnel (10, 20) comporte un domaine supérieur (10a, 20a), un domaine intermédiaire (10b, 20b) et un domaine inférieur (10c, 20c), la matrice (12, 22) ne s'étendant que sur le domaine intermédiaire (10b, 20b).

17. Kit de réactifs selon la revendication 15 ou 16 **caractérisé en ce que** plusieurs récipients réactionnels sont agencés ensemble sur une carte ou une plaque.

18. Kit de réactifs selon l'une des revendications 15 et 16 **caractérisé en ce que** le récipient réactionnel présente un volume de 50 µl à 2 ml.

19. Récipient réactionnel qui contient une matrice poreuse compacte unique où la matrice est agencée de telle manière que, lors d'une centrifugation, il ne se produit pas de déplacement de la matrice à l'intérieur du récipient et que, de préférence, aucun liquide ne peut passer entre la paroi interne du récipient et la matrice.

20. Récipient réactionnel selon la revendication 19 **caractérisé en ce qu'**il comporte un domaine supérieur (10a, 20a), un domaine intermédiaire (10b, 20b) et un domaine inférieur (10c, 20c), la matrice (12, 22) ne s'étendant que sur le domaine intermédiaire (10b, 20b).

21. Récipient réactionnel selon la revendication 19 ou 20 **caractérisé en ce que** le domaine supérieur (20a) comporte un élargissement (26, 78), en particulier sous forme d'un entonnoir ou d'un bombement.

22. Récipient réactionnel selon l'une des revendications 19 à 21 **caractérisé en ce qu'**il présente un volume de 50 µl à 2 ml.

23. Agencement de plusieurs récipients réactionnels ensemble sur une carte ou une plaque **caractérisé en ce que** les récipients réactionnels contiennent une matrice poreuse compacte unique où la matrice est agencée de telle manière que, lors d'une centrifugation, il ne se produit pas de déplacement de la matrice à l'intérieur du récipient et que, de préférence, aucun liquide ne peut passer entre la paroi interne du récipient et la matrice.
